(19) Europäisches Patentamt
European Patent Office
Office européen des brevets

(11) **EP 2 143 696 A1**

(12) **EUROPÄISCHE PATENTANMELDUNG**

(43) Veröffentlichungstag:
**13.01.2010 Patentblatt 2010/02**

(51) Int Cl.:
*C04B 35/622* (2006.01)  *A61C 13/00* (2006.01)
*A61C 13/083* (2006.01)  *A61K 6/02* (2006.01)
*C25D 1/14* (2006.01)  *C04B 35/488* (2006.01)

(21) Anmeldenummer: **09005135.0**

(22) Anmeldetag: **08.04.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **07.06.2008 DE 102008027323**

(71) Anmelder: **Karlsruher Institut für Technologie 76131 Karlsruhe (DE)**

(72) Erfinder:
• **Haußelt, Jürgen**
  **76720 Germersheim (DE)**
• **Binder, Joachim**
  **76227 Karlsruhe (DE)**
• **Pfrengle, Andreas**
  **96120 Bischberg (DE)**

(54) **Verfahren zur Herstellung von keramischen Komponenten**

(57) Verfahren zur Herstellung von keramischen Komponenten, umfassend eine Bereitstellung einer Dispersion mindestens einer ersten und einer zweiten Pulverfraktion aus einer Oxidkeramik sowie einer dritten Pulverfraktion aus einer intermetallischen Verbindung in einer Flüssigkeit, eine Abformung eines Grünkörpers mittels elektrophoretischer Abscheidung aus der Mischung, sowie eine Sinterung des Grünkörpers in einer oxidierenden Atmosphäre zu der keramischen Komponente. Die Aufgabe besteht darin, das Verfahren so zu modifizieren, dass es sich besonders für die Fertigung strukturierter und/oder formsensibler Komponenten eignet. Die Aufgabe wird dadurch gelöst, dass die erste Pulverfraktion durch eine nanoskalige Partikelfraktion mit Partikelgrößen zwischen 2 und 200 nm gebildet wird und als Binder dient, die zweite Pulverfraktion ein Sinteradditiv umfasst, der Anteil der dritten Pulverfraktion in Bezug auf die Summe aller Pulverfraktionen einen Volumenanteil zwischen 50 und 95 % einnimmt sowie die elektrophoretische Abscheidung der Pulverfraktionen zeitgleich erfolgt.

EP 2 143 696 A1

**Beschreibung**

Die Erfindung betrifft ein Verfahren zur Herstellung von keramischen Komponenten gemäß des ersten Patentanspruchs. Das Verfahren dient in seiner Konzeption insbesondere der Herstellung von dichten keramischen Formteilen durch schwindungsreduzierte Sinterung, vorzugsweise einer Reaktionssinterung von Grünkörpern.

Schwindungsreduzierte Sinterungsprozesse zeichnen sich dadurch aus, dass die herzustellende Komponente gegenüber dem Grünkörper nach der Sinterung keine oder nur eine geringe Formänderung aufweist.

Das Prinzip der so genannten Reaktionssinterverfahren beruht auf der Minimierung der beim Sinterprozess auftretenden Schwindung durch volumenvergrößernde Reaktionen, z.B. Oxidationsreaktionen von Metallen und intermetallischen Verbindungen. Am bekanntesten sind hierbei das RBSN- (reaction bonded silicon nitride) und das RBAO-Verfahren (reaction bonded aluminum oxide), die von Silizium bzw. Aluminium ausgehen. Der wesentliche Nachteil dieser zwei Verfahren ist, dass die Keramiken entweder noch Restporosität aufweisen (insbesondere beim RBSN-Verfahren) oder falls es durch bestimmte Varianten, wie SRBSN (Sintered RBSN), es doch gelingt die Keramiken dicht zu sintern, die Schwindung aufgrund der relativ geringen Volumenexpansion nur minimiert und nicht vollständig kompensiert werden kann. Hier haben reaktionsgesinterte Keramiken, die von intermetallischen Verbindungen ausgehen, ihren Vorteil.

Schwindungsreduzierte Sinterverfahren eignen sich besonders für die Herstellung von Prototypen oder von strukturierten und/oder formsensiblen Komponenten, bei denen eine hohe Maßhaltigkeit gefordert wird. Insbesondere bei Mikrobauteilen, bei denen aufgrund der geringen Dimensionen eine Nachbearbeitung nicht möglich ist, zeichnen sich die Reaktionssinterverfahren aus. Aber auch bei der Rekonstruktion von Zähnen oder Zahnkomponenten wie z.B. Kronen oder Füllungen darf sich ein individuell geformter Grünkörper weder bei Anpassungsarbeiten ungewollt verformen noch bei der anschließenden Sinterung vergrößern oder verkleinern.

In der DE 100 49 974 A1 wird ein Verfahren zur Herstellung von keramischen Formteilen für die dentale Anwendung beschrieben. Bei diesem wird zunächst ein Grünkörper abgeformt und anschließend gesintert. Als Ausgangsstoff zur Herstellung des Grünkörpers dient eine Mischung mit einem keramischen und einem metallischen Pulver, die dann zu einem Grünkörper vorzugsweise mittels Elektrophorese abgeformt werden, beispielsweise durch ein elektrophoretisches Abscheideverfahren. Die Sinterung erfolgt unter oxidativen Bedingungen, wobei das metallische Pulver zu Metalloxid oxidiert wird. Dies führt zu einer Minimierung des Sinterschrumpfes. Für die gesinterte keramische Komponente wird eine Dichte von über 90% der theoretischen Dichte angegeben, die für eine dentale Anwendung nicht ausreicht. Folglich wird eine zusätzliche Infiltrierung mit Glaspartikeln zum Verschließen der verbleibenden Porosität vorgeschlagen.

Davon ausgehend liegt die **Aufgabe der Erfindung** darin, ein Verfahren zur Herstellung von keramischen Komponenten so zu modifizieren, dass es sich in besonderem Maße für die Fertigung von strukturierter und/ oder formsensibler Komponenten eignet. Insbesondere sollen diese Komponenten eine hohe Dichte über 95%, vorzugsweise über 97% aufweisen, die eine Verwendung als mechanisch und chemisch stabile Strukturkeramiken z.B. für dentale Anwendungen ermöglicht. Hierbei ist kein nachträgliches Schleißen der Restporosität mit Glaspartikeln mehr notwendig.

Die Aufgabe wird durch ein Verfahren mit den Merkmalen des ersten Patentanspruchs gelöst. Auf diesen rückbezogene Unteransprüche geben vorteilhafte Ausführungsvarianten des Verfahrens wieder.

Die Erfindung basiert auf einem Verfahren zur Herstellung von keramischen Komponenten, umfassend eine Bereitstellung einer Dispersion einer **ersten** und einer **zweiten** sowie in optionalen Ausführungsformen mindestens eine weitere Pulverfraktion aus einer Oxidkeramik sowie einer **dritten** Pulverfraktion aus einer intermetallischen Verbindung in einer Flüssigkeit, einer Abformung eines Grünkörpers mittels elektrophoretischer Abscheidung aus der Mischung sowie einer Sinterung des Grünkörpers in einer oxidierenden Atmosphäre zu der keramischen Komponente.

Durch die Verwendung einer intermetallischen anstelle einer metallischen Pulverfraktion ist es in vorteilhafter Weise möglich, elektrophoretisch abgeschiedene Grünkörper form- und dimensionstreu dicht zu sintern, sodass eine nachträgliche Infiltrierung oder Versiegelung der Restporosität nicht mehr erforderlich ist. Intermetallische Verbindungen sind aufgrund ihrer Werkstoffeigenschaften (spröde, nicht duktil) zwischen Metallen und nicht-metallischen Werkstoffe einzuordnen und bilden damit eine eigene Materialklasse. Während bei Pressformgebungen die fehlende Duktilität der intermetallischen Verbindungen sich nachteilig auf die erzielbaren Gründichten auswirkt, kommt bei der Elektrophorese, bei der die Duktilität keine Rolle spielt, der Vorteil der deutlich größeren relativen Volumenzunahmen während des Reaktionssinterns voll zum tragen. Wesentlich ist auch die in Zusammenhang mit einer simultanen elektrophoretischen Abscheidung der genannten Pulverfraktionen erzielbaren Gründichten und damit die absolute Dimensionstreue des Formkörpers nach der Sinterung.

Die Oxidkeramik umfasst vorzugsweise $ZrO_2$, $Al_2O_3$ und/oder $SiO_2$, die intermetallische Verbindung vorzugsweise $ZrSi_2$ und/oder $ZrAl_3$, die auch als sehr feine Pulver problemlos zu handhaben sind und deren Oxidationsverhalten gut kontrollierbar ist.

Ein Merkmal der Erfindung umfasst die zeitgleiche Abscheidung der intermetallischen und mindestens einer oxidischen Pulverfraktion, im Falle einer wässrigen Lösung als Suspensionsflüssigkeit vorzugsweise in einer neutralen Suspension. Die pH-Werte liegen zwischen 5 und 10, vorzugsweise zwischen 6 und 9. Bei niedrigen pH-Werten ist aufgrund der Zetapotentiale der verwendeten Pulver die Suspensionsstabilität reduziert, bei hohen pH-Werten ist eine zunehmende Blasenbildung im Formkörper zu beobachten, die vermutlich auf eine im Alkalischen stattfindenden Hydroxidbildung nebst Wasserstoffentstehung zurückzuführen ist. Deshalb ist die Verwendung von nahezu neutralen Dispergatoren, wie z.B. Dolapix CE-64, sauren oder basischen Dispergatoren vorzuziehen. CE 64 weist einen pH-Wert von ca. 8 auf. Der eingemischte Dispergatoranteil pro Pulveroberfläche aller eingesetzten Pulverfraktionen beträgt zwischen 200 und 4000 $\mu g/m^2$, vorzugsweise zwischen 250 bis 1000 $\mu g/m^2$, besonders bevorzugt zwischen 300 und 400 $\mu g/m^2$. Auch lässt sich die Wirkung des Dispergators und der neutralen wässrigen Lösung durch die Verwendung einer nicht wässrigen Lösung für die Suspension mit den genannten Partikelfraktionen hervorrufen. Allerdings sind die Abscheideraten in nicht-wässrigen Systemen geringer.

Ein weiteres Merkmal der Erfindung umfasst eine Gestaltung und Verwendung der **ersten** Pulverfraktion als Binder. Die **ersten** Pulverfraktion wird hierzu durch eine nanoskalige Partikelfraktion mit bevorzugten Partikelgrößen zwischen 2 und 200 nm, weiter bevorzugt zwischen 20 und 100 nm gebildet, die vorzugsweise gemeinsam mit der intermetallischen Pulverfraktion abgeschieden wird. Die Partikelgrößen der intermetallischen **dritten** Pulverfraktion liegen zwischen 0,2 und 10 $\mu m$, bevorzugt zwischen 0,5 und 5 $\mu m$, besonders bevorzugt zwischen 0,8 und 3 $\mu m$.

Eine gemeinsame Abscheidung mehrerer Pulverfraktionen, beispielsweise durch eine Membranelektrophorese, bewirkt in vorteilhafter Weise eine homogene Verteilung dieser Pulverfraktionen im Grünkörper. Gerade bei der Verwendung von nanoskaligen Pulverfraktionen wirkt dieses Verfahren einer Agglomeratbildung bei der Verarbeitung entgegen.

Der Anteil der **dritten** Pulverfraktion in Bezug auf die Summe aller Pulverfraktionen nimmt einen Volumenanteil zwischen 50 und 95 % ein. Jeder einzelner prozentualer Volumenanteil in diesem Intervall korreliert mit einer bestimmten reproduzierbaren Sinterschwindung, wobei die von der Zusammensetzung der Suspension oder des Grünkörpers abhängigen Korrelationen über Vorversuche ermittelbar sind.

Vorzugweise weisen die **erste** und **dritte** Pulverfraktion eine bi- oder mehrmodale Größenverteilung auf, d.h. die Partikelgrößen der Pulverfraktionen unterscheiden sich wesentlich, d.h. um mindestens den Faktor 2 voneinander. Insbesondere liegt das bevorzugte Verhältnis der mittleren Partikelgrößen der **dritten** Partikelfraktion zu der ersten zwischen 2,5 und 250, besonders bevorzugt zwischen 5 und 50.

Eine Verwendung von bi- oder mehrmodaligen Pulvermischungen ermöglicht in vorteilhafter Weise hohe Gründichten bei einem Anteil der intermetallischen Pulverfraktion (**dritte** Pulverfraktion) zwischen 50 und 90 Vol.%, bevorzugt zwischen 55 und 80 Vol.% und besonders bevorzugt zwischen 60 und 75 Vol.%. Während bei einem niedrigen Anteil an intermetallischer Phase die hohen Gründichten zwingend erforderlich sind, können bei bestimmten intermetallischen Phasen trotz geringerer Gründichte ein höherer Anteil an intermetallischer Phase von bis zu 95 Vol.% ebenfalls zielführend sein.

Die Gründichten sind nahezu unabhängig von dem Feststoffanteil in der Suspension bei einem Anteil von 15 bis 35 Vol.%. Der Vorteil der Elektrophorese ist, dass dies bis zu einem gewissen Bereich auch für noch geringere Konzentrationen gilt. Durch die Erhöhung des Feststoffgehalts (> 35 Vol.-%) lassen sich die Gründichten nochmals erhöhen; allerdings führt ein zu hoher Feststoffanteil zu einer zu hohen Viskosität, so dass eine sinnvolle Abscheidung nicht mehr möglich ist. Somit sollte der Feststoffanteil in der Suspension zwischen 1 und 60 Vol.% liegen, bevorzugt zwischen 15 und 50 Vol-% und je nach Zusammensetzung besonders bevorzugt zwischen 25 und 35 Vol.%.

Auch für eine schwindungsreduzierte, bevorzugt schwindungsfreie Sinterung ist ein bestimmter Volumenanteil der intermetallischen (**dritten**) Pulverfraktion im Bereich zwischen 40 und 80, bevorzugt zwischen 45 und 70 und weiter bevorzugt zwischen 50 und 65 Vol.% (bei $ZrO_2$, $Al_2O_3$ und/oder $SiO_2$ als die Oxidkeramik und $ZrSi_2$ als intermetallische Verbindung) einzustellen.

Durch eine Einmischung der **zweiten** und ggf. einer **weiteren** Pulverfraktion als Sinteradditiv lässt sich das Sinterverhalten verbessern. Handelt es sich bei dieser zweiten Pulverfraktion auch um ein nanoskaliges Pulver, so wirkt es analog zur ersten Pulverfraktion auch als Bindemittel und verbessert somit auch die Grünkörpereigenschaften, insbesondere die Grünkörperfestigkeit. Der Grünkörper ist damit nicht nur besser entformbar und gegen Beschädigungen resistenter, sondern lässt sich vor einer Sinterung auch gezielt bearbeiten, z.B. durch Fräsen, Drehen oder anderen zerspanende Bearbeitungsverfahren. Vorzugsweise umfasst die **zweite** (und ggf. die **weitere**) Pulverfraktion eine Oxidkeramik, die sowohl als Sinteradditiv die molekularen Transportvorgänge beim Sintern als auch die adhäsiven Eigenschaften im Grünkörper erheblich verbessert. Die **zweite** (und ggf. die **weitere**) Pulverfraktion wird bei der elektrophoretischen Abscheidung und bei der Sinterung vollständig und homogen im Grünkörper bzw. als Werkstoffbestandteil in der keramischen Komponente eingebunden. Der volumenbezogene Anteil der **zweiten** Pulverfraktion am Gesamtanteil der Pulverfraktionen beträgt mindestens 0,5 Vol.%; im Falle einer stofflichen Gleichheit zu einer der **ersten** oder einer **weiteren** oxidkeramischen Pulverfraktion vorzugsweise mindestens 5 Vol.%, weiter bevorzugt mindestens 10 Vol.%, besonders bevorzugt 20%, maximal jedoch bei 50 Vol.%, vorzugsweise 45 Vol.%.

Als Sinteradditiv eignet sich im bevorzugten System $ZrO_2$, $Al_2O_3$ und/oder $SiO_2$ als die Oxidkeramik und $ZrSi_2$ und/oder $ZrAl_3$ als intermetallische Verbindung z.B. MgO, das jedoch eine ausgeprägte Neigung zur Reaktion mit wässrigen Lösungen aufweist. Derartige Reaktionen lassen sich durch eine nichtwässrige Flüssigkeit, z.B. Alkohol als Suspensionsbasis zum Einmischen der Pulverfraktionen vermeiden. Ein als Sinteradditiv bevorzugter $MgAl_2O_4$-Spinell zeigt dagegen bei vergleichbaren Eigenschaften als Sinteradditiv eine wesentlich bessere Reaktionsbeständigkeit in Wasser.

Die Abformung erfolgt bei der Verwendung von wässrigen Systemen bevorzugt durch die Membranelektrophorese, bei der die abzuscheidenden Pulverfraktionen nicht direkt die die Pulverfraktionen anziehenden Elektroden des elektrischen Felds in der Suspension in Kontakt treten. Somit hat die bei der Elektrophorese im wässrigen Medium stattfindende Gasblasenbildung keinen negativen Einfluss auf die Ausbildung des Grünkörpers. Der an einer Membran abgeschiedene Grünkörper lässt sich durch Gegenpolung leichter entformen. Die genannten Erleichterungen reduzieren die für die Entformung erforderlichen auf den Grünkörper einwirkenden mechanischen Kräfte und damit signifikant die Gefahr von Beschädigungen oder Verformungen desselben. Diese Wirkung wird zusätzlich durch eine Trocknung oder Hydrophobisierung der dann besser vom Grünkörper trennbaren Membran unterstützt. Auch ein Trocknen des abgeschiedenen Grünkörpers z.B. im superkritischen Bereich kann zu defektfreien Komponenten führen.

Die Sintertemperaturen liegen bei diesen reaktionsgesinterten Oxidationskeramiken z.B. des Systems $Al_2O_3$-$SiO_2$-$ZrO_2$ je nach Zusammensetzung sowie Art und Menge an Sinteradditiven zwischen 1200 und 1650°C, bevorzugt zwischen 1400 und 1600°C, besonders bevorzugt zwischen 1450-1575°C.

Die Erfindung wird anhand von Ausführungsbeispielen und folgenden Figuren näher erläutert. Es zeigen

**Fig.1** die Partikelgrößenverteilung (Volumenanteil zu Partikeldurchmesser) von beispielhaften $ZrSi_2$-, $ZrO_2$- und $SiO_2$-Suspensionen,

**Fig.2** den Viskositätsverlauf über den Dispergatoranteil bei verschiednen Scherraten in einer rheologischen Untersuchung einer 45 vol.%-igen Suspension (59:31:10 Vol.% $ZrSi_2$:$ZrO_2$:$SiO_2$),

**Fig.3** die Viskosität der Suspension in Abhängigkeit des Feststoffgehalts. Die Zusammensetzung der Pulvermischung ist 59:31:10 Vol.% $ZrSi_2$:$ZrO_2$:$SiO_2$.; die Stabilisierung erfolgt durch TMAH (pH 7,5) bzw. CE 64 (350 $\mu g/m^2$),

**Fig.4a und b** die Zeta-Potentialverläufe von $ZrSi_2$, $ZrO_2$ und $SiO_2$ Suspensionen in Abhängigkeit vom pH-Wert (**a**) und dem Dispergatoranteil (**b**),

**Fig.5** den schematischen Aufbau der Zelle zur elektrophoretischen Abscheidung mittels Membranelektrophorese in einer Explosionszeichnung,

**Fig.6** die relativen Gründichten elektrophoretisch abgeschiedener Formkörper als Funktion der Grünkörperzusammensetzung,

**Fig.7** die relative Gründichten in Abhängigkeit des Feststoffgehalts,

**Fig.8** die Viskosität von 35 vol.%-igen Suspensionen mit unterschiedlichem Zusammensetzungen ($ZrSi2 : ZrO2 : SiO2 : MgAl_2O_4$) sowie

**Fig.9** die Zeta-Potentiale von Suspensionen mit alternative intermetallische Pulverfraktionen in Abhängigkeit zu den pH-Werten in analoger Weise zu **Fig.4a**

**Ausführungsbeispiel:**

Für eine elektrophoretische Abscheidung von Grünkörpern wurden mehrere Suspensionen mit Pulverfraktionen mit den folgend genannten Feststoffkomponenten (Stoffe) hergestellt:

- $ZrSi_2$ (Fa. H.C. Starck)
- $ZrO_2$, TZ-0 (monoklin), (Fa. Tosoh)
- $SiO_2$, Aerosil OX 50 (Fa. Degussa)
- MgO (Fa. Merck)
- $Al_2O_3$, $\gamma$-Tonerde (Fa. Merck)
- $MgAl_2O_4$ Magnesium-Aluminum-Oxid 99,985% (Fa. Alfa Aesar)

Für die Herstellung der Suspensionen werden vorwiegend $ZrSi_2$, $ZrO_2$ und optional das nanoskalige $SiO_2$ als anorganischer Binder eingesetzt, die restlichen Stoffe dienen als Sinteradditive. **Tab.1** gibt die Dichten und die Molmassen der vorgenannten Feststoffkomponenten wieder. Die Pulverfraktionen werden in deionisiertem Wasser (DI-Wasser) dispergiert. Die Stabilisierung der Suspensionen wird entweder durch Zugabe von Tetramethylammoniumhydroxid (TMAH, 25 Gew.-% Lösung in Wasser, Fa. Sigma Aldrich) oder durch DOLAPIX CE 64 (Fa. Zschimmer & Schwarz) als Dispergator erreicht, der eine Aktivsubstanzkonzentration von 65 % und eine Dichte von 1,2 g/cm$^3$ besitzt.

**Tab.1:** Stoffdaten der verwendeten Ausgangsstoffe.

|  | ZrSi$_2$ | ZrO$_2$ | SiO$_2$ | MgO | Al$_2$O$_3$ | MgAl$_2$O$_4$ |
|---|---|---|---|---|---|---|
| **Dichte (g/cm$^3$)** | 4,88 | 5,83 | 2,20 | 3,58 | 3,94 | 3,60 |
| **Molmasse (g/mol)** | 147,40 | 123,22 | 60,08 | 40,30 | 101,96 | 142,27 |

Das herangezogene ZrSi$_2$ besitzt im Anlieferungszustand eine BET-Oberfläche von 1,83 m$^2$/g, eine mittlere Partikelgröße d$_{50}$ von 3,02 $\mu$m und eine Massenzunahme von 62,70 %. Gemäß Analysezertifikat besteht das Pulver zu 37,5 Gew.-% aus Si, 0,8 Gew.-% aus O, 0,16 Gew.-% aus C, 0,13 Gew.-% aus N und 0,083 Gew.-% aus Fe.

Das ZrSi$_2$ wird in einer Rührwerkskugelmühle in Isopropanol aufgemahlen. Der mit ZrO$_2$ ausgekleidete Rührbehälter ist mit 0,8 mm durchmessenden, mit Y$_2$O$_3$ stabilisierten ZrO$_2$-Mahlkugeln gefüllt. Die im Rahmen des Ausführungsbeispiels hergestellten ZrSi$_2$-Pulverchargen für die Grünkörperherstellung werden im Folgenden mit FM017 und FM018 bezeichnet.

Bei der Grünkörperherstellung kommen ebenfalls zwei ZrO$_2$-Chargen zum Einsatz (Z001889P und Z005551P), deren spezifische Oberflächen ebenfalls bestimmt wurden.

Die thermogravimetrisch bestimmte Massenzunahme des ZrSi$_2$-Pulvers vor der Mahlung beträgt theoretisch 65,13 %. Zudem wird das Pulver vor der Verwendung für die elektrophoretischen Abscheidung für eine Stunde bei 400 °C getempert (Heizrate 5 K/min, Abkühlrate 10 K/min), um organische Rückstände zu entfernen.

Vor Einmischung der Pulverfraktionen in eine vorbereitete und mit Dispergator bzw. einer TMAH-Base (TMAH = Tetramethylammoniumhydroxid) versehenen Flüssigkeit (Dispergiermedium DI-Wasser) zu einer Suspension wurden diese gewogen und anschließend vor der Eingabe trocken gemischt. Die Zugabe der Pulverfraktionen in die Flüssigkeit erfolgt, je nach Feststoffgehalt, in einem Zeitraum von 2 bis 15 Minuten. Anschließend wird die Suspension vorzugsweise etwa 30 Minuten gerührt, gefolgt von einer weiteren Dispergierung mittels Ultraschall. Aufgrund der dissipierten Ultraschallleistung wird die Suspension zur Vermeidung von Erwärmung in einem Eiswasserbad gekühlt.

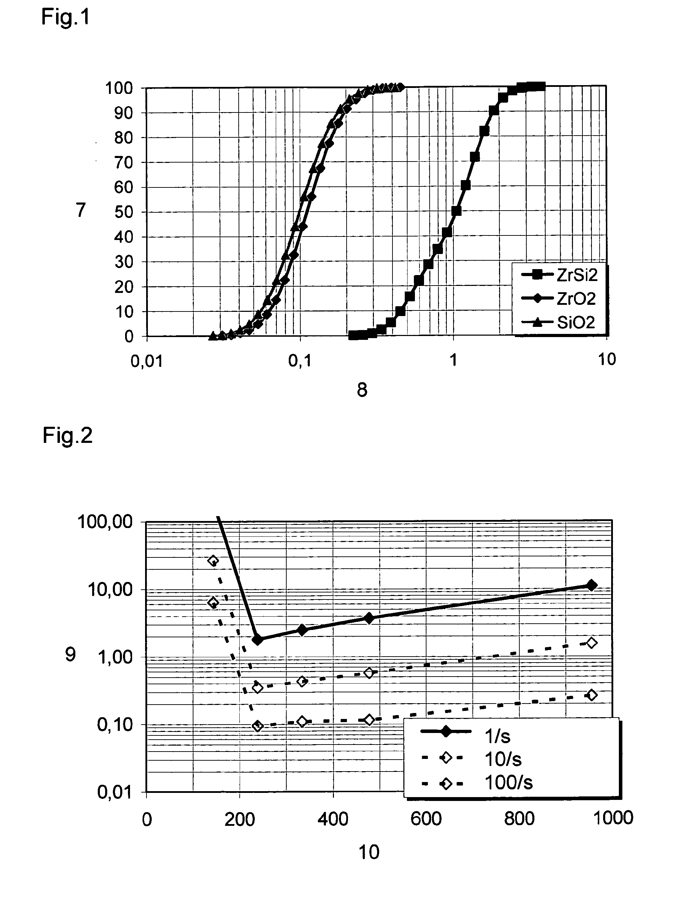

In **Fig.1** sind die Partikelgrößenverteilungen (Volumenanteil **7** in [%] über die Partikeldurchmesser **8** in [$\mu$m]) CE 64-stabilisierter, einphasiger ZrSi$_2$ (Zirkoniumdisilizid), ZrO$_2$ (Zirkoniumoxid) und SiO$_2$-Suspensionen (Siliziumdioxid, nanoskalig) dargestellt (akustisches Messverfahren). Die ZrSi$_2$-Pulverfraktion repräsentiert die dritte Pulverfraktion, ZrO$_2$ und SiO$_2$-Pulverfraktionen die erste und ggf. eine weitere Pulverfraktion. Die ZrSi$_2$- und ZrO$_2$-Suspensionen für das akustische Messverfahren sind mit 35 Vol.-% Feststoffgehalt hergestellt, die SiO$_2$-Suspension aufgrund der schwierigeren Dispergierbarkeit des Pulvers lediglich mit dem halben Feststoffgehalt (17,5 Vol.%). Der Dispergatorgehalt der für die PSD-Messungen verwendeten Suspensionen wird so hoch gewählt, dass auf jeden Fall genügend Dispergator für eine vollständige Oberflächenbelegung der Partikel vorhanden ist. Die d$_{50}$-Werte liegen für ZrO$_2$ und SiO$_2$ etwa bei 100 bis 130 nm und für ZrSi$_2$ bei 1,1 $\mu$m, die d$_{90}$-Werte bei etwa 200 nm für SiO$_2$ und etwa 2 $\mu$m für ZrSi$_2$. Ein d$_{90}$-Wert für das ZrO$_2$ ist aufgrund der unterschiedlichen Messdaten nicht eindeutig bestimmbar. In etwa kann jedoch gesagt werden, dass die Partikel des ZrSi$_2$-Pulvers um den Faktor 10 größer sind als die der anderen beiden Pulver.

Die Bestimmung des optimalen Dispergatorgehalt der Suspensionen erfolgt durch rheologische Untersuchungen. **Fig.2** zeigt die Viskosität **9** in [Pas] über den Dispergatoranteil CE-64 **10** in [$\mu$m/m$^2$] bei verschiedenen Scherraten 1/s, 10/s und 100/s. Für die Messungen wurden Mischungen der drei Pulverfraktionen herangezogen. Die Messungen werden, um einen deutlichen Einfluss auf die Viskosität zu sehen, an 45 vol.%igen Suspensionen durchgeführt. Die Suspensionen bestehen zu 59:31:10 Vol.% aus ZrSi$_2$:ZrO$_2$:SiO$_2$. Vorzugsweise beträgt die Oberflächenbelegung bei dieser Mischung mindestens 250 $\mu$g/m$^2$. Ein höherer Dispergatorgehalt führt zu einer geringfügig höheren Viskosität. Um stets, d.h. auch im Falle von Entmischungen einen ausreichenden Dispergatoranteil sicherzustellen, werden ein Dispergatoranteil von 300 bis 400 $\mu$g/m$^2$ bevorzugt. Dies entspricht im Falle der gegebenen Mischung einem auf die Pulvermasse bezogenen Dispergatorgehalt von 0,35 Gew.-%.

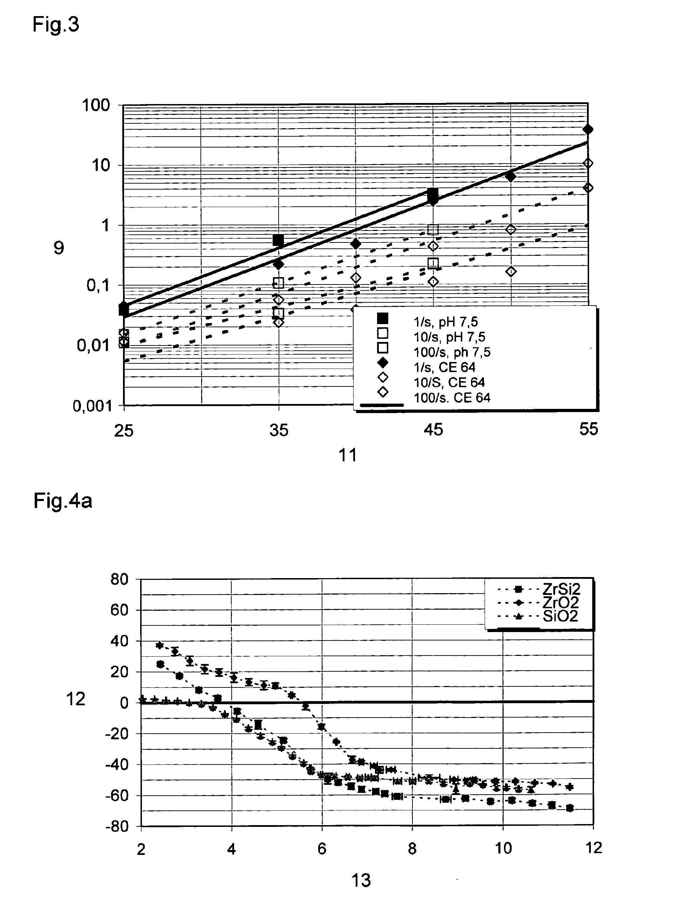

**Fig.3** gibt die Viskosität **9** in [Pas] über den Feststoffgehalt **11** in [Vol.%] bei verschiedenen Scherraten 1/s, 10/s und 100/s wieder. Die Viskosität einer Suspension nimmt mit steigendem Feststoffgehalt exponentiell zu. Neben der Stabilisierung mit CE 64 ist hier auch die Stabilisierung mit einer geringen Menge TMAH bei einem pH-Wert von 7,5 gegeben. Beim Vergleich der Viskositäten wird ersichtlich, dass die Stabilisierung mit TMAH teils zu deutlich höheren Viskositäten führt als mit CE 64.

Neben der Partikelgrößenverteilung und den rheologischen Eigenschaften der Suspension ist das Zeta-Potential der Partikel von großer Bedeutung. **Fig.4a** gibt das Zeta-Potential **12** in [mV] über den pH-Wert **13** ohne Zugabe eines Disperators wieder. Während im sauren Bereich ein schwach positives Zeta-Potential vorliegt, befindet es sich ab einem pH-Wert von etwa 7 relativ stabil zwischen -40 und -60 mV. Die isoelektrischen Punkte liegen bei etwa 3, 4 und 5,5 für $SiO_2$, $ZrSi_2$ und $ZrO_2$.

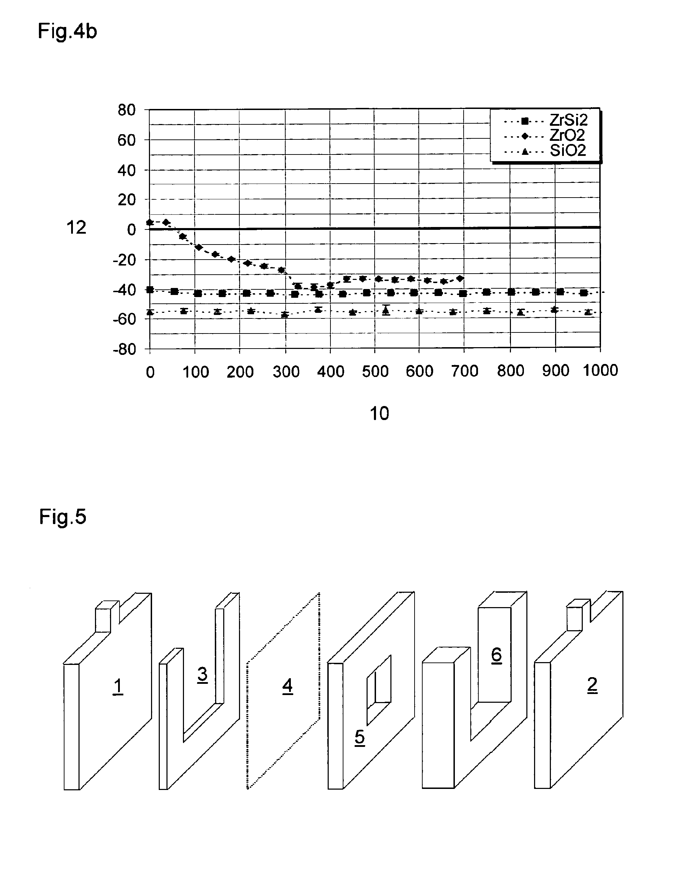

**Fig.4b** zeigt dagegen den Verlauf des Zeta-Potentials **12** in [mV] über den Dispergatorgehalt CE-64 **10** in [$\mu$g/m²]. Sowohl $ZrSi_2$ als auch $SiO_2$ weisen ohne Zugabe eines Dispergators bereits ein negatives Zeta-Potential auf, während $ZrO_2$ eine gewisse Menge von mindestens 100, vorzugsweise 350 $\mu$g/m² an Dispergator zur Sicherstellung eines negativen Zeta-Potentials benötigt. Der Dispergator lagert sich an die Oberfläche an und verändert damit die Oberflächenladung und somit das Zetapotential. Die pH-Werte der hochverdünnten Suspensionen ohne Dispergator betragen etwa 6 bis 7.

Weiterhin werden die pH-Werte und die spezifischen elektrischen Leitfähigkeitswerte der Suspensionen gem. den Partikelgrößenverteilungen aus **Fig.1** in **Tab.2** protokolliert. Zusätzlich finden sich dort die charakteristischen Werte für eine Suspension einer Mischung, die zu nahezu schwindungsfreien Formkörpern führt. Diese besteht aus 61:32:5: 2 Vol.% $ZrSi_2$:$ZrO_2$:$SiO_2$:$MgAl_2O_4$. Bei dieser Suspension sind Mittelwert und Standardabweichung fünf verschiedener Suspensionen ähnlicher (nicht identischer) Zusammensetzung angegeben. Zu beachten gilt außerdem, dass besonders die $SiO_2$-Suspension aufgrund der hohen BET-Oberfläche viel Dispergator enthält.

**Tab.2:** pH-Werte und Leitfähigkeiten. *Schwindungsfrei* besteht aus 61:32:5:2 Vol.% $ZrSi_2$:$ZrO_2$:$SiO_2$:$MgAl_2O_4$.

|  | **pH-Wert** | **Leitfähigkeit ☐ (S/m)** |
|---|---|---|
| **35 Vol.-% $ZrSi_2$** | 7,52 | 0,1084 |
| **35 Vol.-% $ZrO_2$** | 8,53 | 0,1450 |
| **17,5 Vol.-% $SiO_2$** | 8,68 | 0,3003 |
| **Schwindungsfrei** | 8,06 ± 0,30 | 0,1071 ± 0,0100 |

Eine Zusammenstellung aller im Rahmen der im Folgenden beschriebenen Ausführungen zur Gründichte hergestellten Suspensionen ist nachfolgend in **Tab.3** wiedergegeben.

**Tab.3** Zusammensetzung und molares Verhältnis der untersuchten Suspensionen sowie der Gründichten der aus diesen hergestellten Grünkörper

| Nr. | **Zusammensetzung** | | | | **Molares Verhältnis Zr/Si** | **Gründichte** | | | |
|---|---|---|---|---|---|---|---|---|---|
| | **Fest-stoff (Vol.-%)** | **$ZrSi_2$ (Vol.- %)** | **$ZrO_2$ (Vol.-%)** | **$SiO_2$ (Vol.-%)** | | **Theoretisch (g/cm³)** | **absolut (g/cm³)** | **Relativ (%)** | **St.Abw.** |
| 1 | 35% | 0% | 100% | 0% | inf | 5,830 | 3,267 | 56,04% | 0,24% |
| 2 | 35% | 30% | 70% | 0% | 2,170 | 5,546 | 3,366 | 60,68% | 0,04% |
| 3 | 35% | 50% | 50% | 0% | 1,214 | 5,356 | 3,355 | 62,64% | 0,13% |
| 4 | 35% | 60% | 40% | 0% | 0,976 | 5,262 | 3,348 | 63,63% | 0,09% |
| 5 | 35% | 65% | 35% | 0% | 0,885 | 5,215 | 3,326 | 63,78% | 0,17% |
| 6 | 35% | 70% | 30% | 0% | 0,806 | 5,167 | 3,303 | 63,92% | 0,22% |
| 7 | 35% | 75% | 25% | 0% | 0,738 | 5,120 | 3,259 | 63,65% | 0,13% |
| 8 | 35% | 80% | 20% | 0% | 0,679 | 5,073 | 3,186 | 62,81% | 0,17% |
| 9 | 35% | 90% | 10% | 0% | 0,579 | 4,977 | 3,040 | 61,08% | 0,17% |
| 10 | 35% | 100% | 0% | 0% | 0,500 | 4,883 | 2,878 | 58,93% | 0,08% |
| 11 | 35% | 0% | 90% | 10% | 11,630 | 5,467 | 3,087 | 56,47% | 0,36% |
| 12 | 35% | 30% | 60% | 10% | 1,628 | 5,183 | 3,087 | 59,56% | 0,17% |
| 13 | 35% | 50% | 40% | 10% | 0,965 | 4,994 | 3,114 | 62,37% | 0,05% |
| 14 | 35% | 60% | 30% | 10% | 0,785 | 4,899 | 3,103 | 63,35% | 0,11% |

(fortgesetzt)

| Nr. | Zusammensetzung | | | | Molares Verhältnis Zr/Si | Gründichte | | | |
|---|---|---|---|---|---|---|---|---|---|
| | Fest-stoff (Vol.-%) | ZrSi₂ (Vol.- %) | ZrO₂ (Vol.-%) | SiO₂ (Vol.-%) | | Theoretisch (g/cm³) | absolut (g/cm³) | Relativ (%) | St.Abw. |
| 15 | 35% | 70% | 20% | 10% | 0,653 | 4,805 | 3,083 | 64,17% | 0,05% |
| 16 | 35% | 80% | 10% | 10% | 0,551 | 4,709 | 3,002 | 63,74% | 0,05% |
| 17 | 35% | 90% | 0% | 10% | 0,471 | 4,615 | 2,880 | 62,41% | 0,35% |
| 18 | 35% | 0% | 80% | 20% | 5,170 | 5,104 | 2,860 | 56,03% | 0,16% |
| 19 | 35% | 24% | 56% | 20% | 1,485 | 4,877 | 2,819 | 57,80% | 0,10% |
| 20 | 35% | 48% | 32% | 20% | 0,793 | 4,649 | 2,857 | 61,45% | 0,02% |
| 21 | 35% | 56% | 24% | 20% | 0,673 | 4,574 | 2,892 | 63,23% | 0,14% |
| 22 | 35% | 64% | 16% | 20% | 0,579 | 4,498 | 2,887 | 64,17% | 0,24% |
| 23 | 35% | 72% | 8% | 20% | 0,502 | 4,422 | 2,865 | 64,80% | 0,13% |
| 24 | 35% | 80% | 0% | 20% | 0,439 | 4,346 | 2,825 | 64,99% | 0,33% |
| 25 | 15% | 70,4% | 29,6% | 0% | 0,800 | 5,163 | 3,307 | 64,05% | 2,25% |
| 26 | 25% | 70,4% | 29,6% | 0% | 0,800 | 5,163 | 3,252 | 62,98% | 0,22% |
| 27 | 35% | 70,4% | 29,6% | 0% | 0,800 | 5,163 | 3,238 | 62,71% | 0,22% |
| 28 | 40% | 70,4% | 29,6% | 0% | 0,800 | 5,163 | 3,324 | 64,38% | 0,11% |
| 29 | 45% | 70,4% | 29,6% | 0% | 0,800 | 5,163 | 3,420 | 66,24% | 0,05% |
| 30 | 50% | 70,4% | 29,6% | 0% | 0,800 | 5,163 | 3,442 | 66,67% | 0,17% |
| 31 | 15% | 59,0% | 31,0% | 10% | 0,800 | 4,908 | 3,127 | 63,71% | 0,17% |
| 32 | 25% | 59,0% | 31,0% | 10% | 0,800 | 4,908 | 3,110 | 63,36% | 0,37% |
| 33 | 35% | 59,0% | 31,0% | 10% | 0,800 | 4,908 | 3,116 | 63,49% | 0,15% |
| 34 | 40% | 59,0% | 31,0% | 10% | 0,800 | 4,908 | 3,166 | 64,51% | 0,09% |
| 35 | 45% | 59,0% | 31,0% | 10% | 0,800 | 4,908 | 3,242 | 66,06% | 0,53% |
| 36 | 50% | 59,0% | 31,0% | 10% | 0,800 | 4,908 | 3,249 | 66,19% | 0,17% |
| 37 | 55% | 59,0% | 31,0% | 10% | 0,800 | 4,908 | 3,238 | 65,98% | 0,83% |

Die elektrophoretische Abscheidung erfolgte als Membran-Elektrophorese. **Fig.5** gibt im Rahmen des Ausführungsbeispiels einen möglichen schematischen Aufbau der Zelle zur elektrophoretischen Abscheidung in einer Explosionszeichnung wieder. Zwischen einer Anode **1** und einer Kathode **2** sind ausgehend von der Anode eine Ausgleichskammer **3** (Kunststoff, Schichtdicke ca. 2 mm), eine Membran **4,** ein Formfenster **5** (Kunststoff, Schichtdicke ca. 5 mm), eine Suspensionskammer **6** (Kunststoff, Schichtdicke ca. 10 mm) schichtweise angeordnet. Das Formfenster für die Abscheidung des Grünkörpers misst im Ausführungsbeispiel ca. 3 bis 5 cm².

Für die Membranen eignen sich vorzugsweise regenerierbare Zelluloseflächen (Wandstärke z.B. 20 bis 50 $\mu$m), wie sie z.B. aus der Dialysetechnik bekannt sind. Neben der Verwendung folienartiger, unstrukturierter Membranen werden auch formstabile unstrukturierte und strukturierte Membranen unterschiedlicher Materialien vorgeschlagen. Diese werden vorzugsweise durch Gießen und Aushärten einer flüssigen Mischung folgender Materialien hergestellt:

- PMMA-MMA: Polymethylmethacrylat-Partikel (Scientific Polymer 037D, MW 540.000, $d_{50}$ = 90 $\mu$m) werden mit Methylmethacrylat (MMA, Fa. Sigma Aldrich), DI-Wasser und Walloxen NO 40 (Nonylphenolethoxylat mit 4 Ethylenoxidgruppen, Fa. Wall Chemie) gemischt und gegossen, so dass bei Polymerisation ein poröses Gerüst entsteht.
- Deguvest CF (Fa. Degussa): Phosphatgebundene Einbettmasse $Mg_3(PO_4)_2$, die mit spezieller Anmischflüssigkeit angerührt wird und aushärtet.
- Superhartgips (Fa. Wieland Dental + Technik): Dentalgips, der mit DI-Wasser gemischt wird und anschließend aushärtet.

Die größere der beiden Kammern der Zelle, umfassend die Suspensionskammer **6** und das Formfenster **5** wird mit der abzuscheidenden Suspension gefüllt, die kleinere Ausgleichskammer **3** mit einer Ausgleichsflüssigkeit bestehend aus verdünnter, wässriger TMAH-Lösung (Tetramethylammoniumhydroxid-Lösung). Das Leitfähigkeitsverhältnis von Ausgleichsflüssigkeit zu Suspension ist dabei auf einen bestimmten Wert, vorzugsweise 10 einzustellen. Dies erfolgt vorzugsweise über eine TMAH-Zugabe zur Ausgleichsflüssigkeit. Die Membran **4** wurde zur Vermeidung von unerwünschten Gasreservoirs mit DI-Wasser infiltriert.

Die an den Elektroden **1, 2** anliegende elektrische Feldstärke während der Abscheidung wurde im Bereich zwischen 5 und 25 V/cm eingestellt. Die Dauer der Abscheidung beläuft sich auf 1 bis 5 min für Abscheidungen auf einer ebenen Membran und erhöht sich auf bis zu 10 min bei Abscheidungen auf strukturierten Membranen. Danach wird die Zelle zerlegt und der Grünkörper aus dem Formfenster entnommen.

Bei der Abscheidung ist die exakte Einhaltung des pH-Wertes insbesondere aus TMAH-stabilisierten Suspensionen zwingend erforderlich. Mit steigenden pH-Werten ist eine zunehmende Blasenbildung im Formkörper zu beobachten. Bei pH-Werten kleiner als 7 reduziert sich dagegen die Stabilität der Suspension und damit die Effizienz und Durchführbarkeit der Abscheidung. Daher haben sich für TMAH-stabilisierte Suspensionen pH-Werte zwischen 7 und 8 als am besten geeignet erwiesen.

Eine alternative Stabilisierung wurde durch einen zitronensäurehaltigen Dispergator erzielt. Bei ausreichendem Dispergatorgehalt ist eine zusätzlich pH-Wertänderung zur Stabilisierung nicht erforderlich. Der optimale Dispergatorgehalt wurde anhand rheologischer Messungen zwischen 300 und 400, vorzugsweise zu etwa 350 $\mu$g pro m$^2$ Partikeloberfläche bestimmt.

Eine Stabilisierung mit CE-64 erfordert dagegen in vorteilhafter Weise keine exakte Einhaltung eines bestimmten pH-Wertebereichs.

Ferner wurden wässrige keramische Suspensionen unterschiedlicher Zusammensetzung, bestehend aus Zirkoniumdisilizid, Zirkoniumoxid und einem nanoskaligen Siliziumoxid als anorganischer Binder hergestellt und elektrophoretisch abgeschieden. Die Zugabe von nanoskaligem $SiO_2$ führte zu einer erhöhten Festigkeit des Grünkörpers, die zwar bevorzugt ist und das Handling der Grünkörper erleichtert, aber nicht zwingend erforderlich ist. An den abgeschiedenen Grünkörpern wurde anschließend die Gründichte bestimmt. Dabei wurde sowohl ihre Abhängigkeit von der Materialzusammensetzung als auch vom Feststoffgehalt der Suspension ermittelt.

Die Gründichten für die im Rahmen der Ausführung offenbarten Suspensionszusammensetzungen sind wie diese in **Tab.3** wiedergegeben.

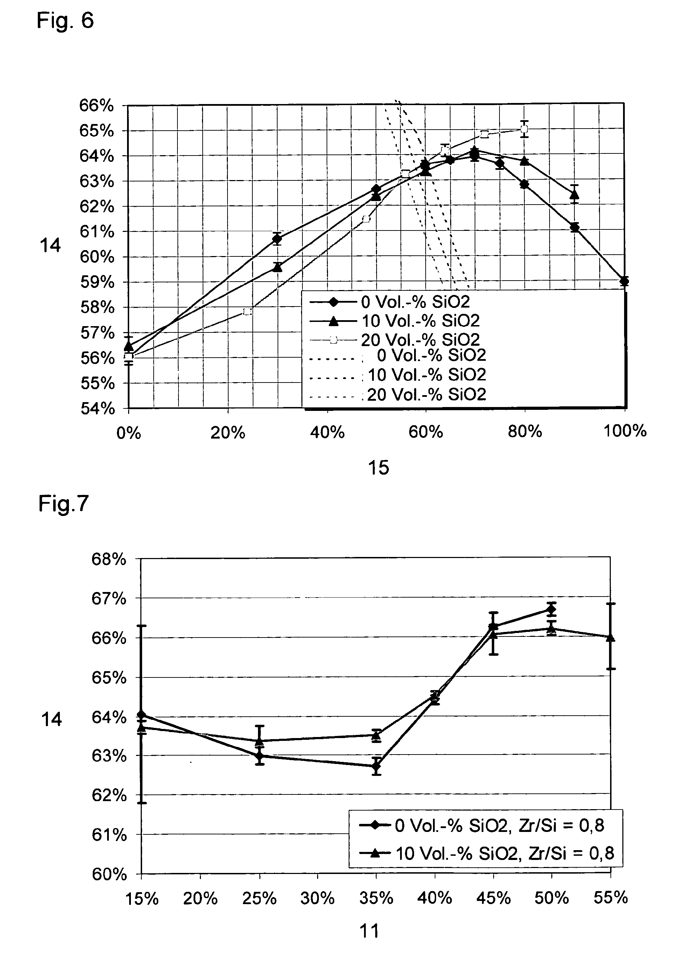

Die Abhängigkeit der Gründichte von den einzelnen Faktoren ist nachfolgend für eine 35 vol.%-ige Suspensionen in **Fig.6** graphisch dargestellt. **Fig.6** zeigt die relativen Gründichten **14** in [% theoretische Dichte] bezogen auf den Volumenanteil **15** in [%] von $ZrSi_2$ zu $ZrSi_2$ + $ZrO_2$ + 0, 10 und 20 Vol.% Si02 (d.h. nicht auf den gesamten Feststoffgehalt). Ebenso eingezeichnet sind für den jeweiligen $SiO_2$-Gehalt die erforderlichen Gründichten für schwindungsfreies Sintern bei gegebener Zusammensetzung der Suspension (Legende: "DV=0"). Diese Kurven wurden anhand der nach dem Sintern erwartungsgemäß vorhandenen Phasenanteile und ihrer Dichten analytisch berechnet. Für schwindungsfreies Sintern kommen somit nur diejenigen Materialzusammensetzungen in Frage, bei denen sich die Kurven von gemessener Gründichte und erforderlicher Gründichte schneiden. Diese liegen in diesem Beispiel bei Mischungsverhältnissen $ZrSi_2$/$ZrO_2$/$SiO_2$ von etwa:

- 60 / 40 / 0 Vol.%

- 58 / 32 / 10 Vol.%

- 56 / 24 / 20 Vol.%

Weiterer Spielraum bei der Variation des Materials kann in geringem Maße durch eine Anpassung des Feststoffgehalts erfolgen. **Fig.7** zeigt die relative Gründichten **14** in [% theoretische Dichte] in Abhängigkeit des Feststoffgehalts **11** in [Vol.%]. Bei Feststoffgehalten unterhalb von 35 Vol.% verläuft die Gründichte, beispielhaft ermittelt für Grünkörper mit einem Zr/Si-Verhältnis von 0,8, im Bereich von 63...64 % der theoretischen Dichte. Bei einer Steigerung des Feststoffgehaltes auf mindestens 40, vorzugsweise 45 bis 55 oder 60 Vol.% stellt sich eine erhöhte und damit bevorzugte Gründichte von etwa 66 % der theoretischen Dichte ein.

Um festzustellen, ob die Zusammensetzung der Grünkörper auch der Zusammensetzung der Suspension entspricht, wurde der $ZrSi_2$-Gehalt gravimetrisch ermittelt. Dazu wurden abgeschiedene Formkörper fein gemörsert und das Pulver anschließend für 4 Stunden bei 1600 °C oxidiert. Durch die Massenzunahme lässt sich auf den $ZrSi_2$-Gehalt schließen, der in allen Fällen im Rahmen des Messfehlers bei 100 $\pm$ 1 % des $ZrSi_2$-Gehaltes der Suspension lag.

Eine Entformung aus strukturierten Membranen erfolgt vorzugsweise nach einer kurzzeitigen Gegenpolung zwischen den Elektroden **1, 2** mit vorzugsweise gleicher, aber umgekehrt gepolter elektrischer Feldstärke, wodurch sich der Formkörper auf der Membran lockert und leichter von dieser entfernbar ist. Alternativ erleichtert eine vorangegangene Trocknung (z.B. durch konvektive Aufheizung und/oder Unterdruck) des Grünkörpers noch im Formfenster 4 eine Entformung. Durch eine hängende Lage kann sich der Formkörper durch sein Eigengewicht entformen. Auch eine Hydrophobisierung einer vorzugsweise strukturierten Membranoberfläche, z.B. durch einen dünnen Paraffinfilm, erschwert eine adhäsive Anbindung des Grünkörpers und erleichtert damit die Entformung.

Die elektrophoretisch abgeschiedenen Grünkörper wurden im Rahmen des Ausführungsbeispiels nach der Entformung oxidiert und gesintert. Das Temperaturprogramm wurde dabei so angepasst, um im unteren Temperaturbereich eine vollständige Oxidation und im oberen Temperaturbereich eine gute Sinterung gewährleistet ist. Als geeignetes, zeitlich nicht optimiertes Temperaturprogramm hat sich für die Oxidation das folgende ergeben:

$$0 \xrightarrow{5} 480 \xrightarrow{1,2} 600 \xrightarrow{0,25} 650 \xrightarrow{0,15} 760 \xrightarrow{1} 840 \xrightarrow{4} 1100, 16\,h \qquad (1)$$

Das Programm sieht einen Temperaturanstieg mit unterschiedlichen Heizraten (über den Pfeilen angegebene Zahlen in [K/min]) über verschiedene Zwischentemperaturen (in [°C]) bis auf 1100 °C vor. Diese Temperatur wird 16 Stunden lang gehalten, bevor die Sinterung mit dem folgenden Sinterprogramm eingeleitet wird:

$$1100 \xrightarrow{5} 1275, 16\,h \xrightarrow{5} 1300, 16\,h \xrightarrow{5} 1325, 16\,h \xrightarrow{5} 1575, 4\,h \quad (2)$$

Am Ende der vierstündigen Haltezeit wird mit der sich zu einem Sinterkörper verwandelte Grünkörper mit einer Abkühlungsrate von 10 K/min auf Raumtemperatur abgekühlt.

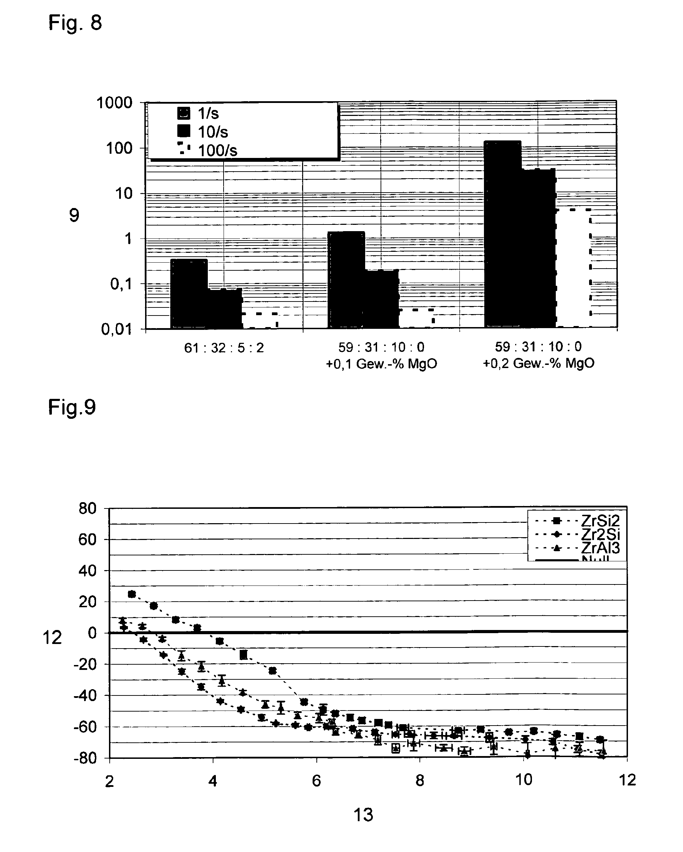

Für eine zuverlässige Sinterung der Grünkörper ist die Zugabe von Sinteradditiven erforderlich. Erfahrungsgemäß eignen sich hierzu insbesondere Aluminiumoxid und Magnesiumoxid. Bei der Herstellung MgO-haltiger wässriger Suspensionen zeigte sich bereits ab einer zugegebenen Menge von etwa 0,1 Gew.% MgO jedoch nach kurzer Zeit eine rasche Zunahme der Viskosität, die eine elektrophoretische Abscheidung unmöglich macht. **Fig.8** zeigt hierzu die Viskosität **9** in [Pas] in Abhängigkeit der Zusammensetzungen (ZrSi2 : ZrO2 : SiO2 : MgAl$_2$O$_4$) **16** in [Vol.%] bei geringer Scherrate, wie sie für elektrophoretische Abscheidungen relevant ist. Die beiden rechts dargestellten Zusammensetzungen beinhalten zusätzlich 0,1 bzw. 0,2 Gew.% MgO.

Bei der Verwendung von Spinell (MgAl$_2$O$_4$) tritt dieser viskositätssteigernde Effekt nicht auf. Es wurden Suspensionen mit bis zu 2 Vol.% Spinell (**Fig.8**, links dargestellte Zusammensetzung) ohne den vorgenannten Effekt elektrophoretisch abgeschieden und gesintert.

Nach dem Sintern erfolgte eine Bestimmung der Volumenänderung gegenüber dem Grünkörper. Zum einen geht dies über die Kenntnis der absoluten Massenzunahme des ZrSi$_2$, dessen Anteil im Formkörper, sowie die absoluten Grün- und Sinterdichten. Zum anderen wurden aus Grünkörpern planparallele Scheiben geschnitten, deren Längen im Grün- und Sinterzustand verglichen wurden. Dabei wurde an einem als rechnerisch schwindungsfrei ermittelten Formkörper eine verbleibende Längenzunahme von 1,07 % mit einer Standardabweichung von 0,57 % (aus 5 verschiedenen Proben) ermittelt.

**Tab.4** gibt die empirisch gemessenen relativen Längenänderurigen (Sinterschwindungen) von Sinterprobekörpern der Zusammensetzung 55:33:10:2 Vol.-% (ZrSi$_2$:ZrO$_2$:SiO$_2$:MgAl$_2$O$_4$) in unterschiedlichen Messrichtungen wieder. Die Sinterprobekörper weist hierfür Oberflächenstrukturen mit quaderförmigen oder würfelförmigen Strukturen auf, die in ihrer Kantenlänge (Kanten), Diagonalen und Höhe (Vertikale) zuverlässig messbar sind. Der untersuchte Formkörper ist bei der gegebenen Materialzusammensetzung im Rahmen der Messgenauigkeit schwindungsfrei. Die Standardabweichungen sind jedoch, besonders bei der vertikalen Messung, sehr hoch.

| **Tab.**Fehler! Kein Text mit angegebener Formatvorlage im Dokument.: Relative Längenänderung von Sinterkörper im Vergleich zum Grünkörper der Zusammensetzung 55:33:10:2 Vol.-% (ZrSi$_2$:ZrO$_2$:SiO$_2$:MgAl$_2$O$_4$) in unterschiedlichen Messrichtungen. | |
| --- | --- |
| **Messrichtung** | **Längenänderung** |
| Kanten | 0,21 % $\pm$ 0,62% |
| Diagonal | 0,08 % $\pm$ 0,62 % |

(fortgesetzt)

| Tab.Fehler! Kein Text mit angegebener Formatvorlage im Dokument.: Relative Längenänderung von Sinterkörper im Vergleich zum Grünkörper der Zusammensetzung 55:33:10:2 Vol.-% ($ZrSi_2$:$ZrO_2$:$SiO_2$:$MgAl_2O_4$) in unterschiedlichen Messrichtungen. | |
|---|---|
| **Messrichtung** | **Längenänderung** |
| Vertikal | 0,15 % $\pm$ 1,48 % |

Neben Wasser wurde Ethanol als mögliches Dispergiermedium (Flüssigkeit als Basis für die Suspension) untersucht. Hierbei zeigte sich jedoch bei einer 35 vol%igen Suspension eine sehr hohe Viskosität. Sie lag bei einer Scherrate von 1/s bereits deutlich über 100 Pas, was für eine elektrophoretische Abscheidung nicht mehr tolerierbar ist. Für die Stabilisierung wurden dabei sowohl der eingangs erwähnte zitronensäurehaltige Dispergator, als auch eine Trioxidekansäure untersucht.

Die Erfindung schließt grundsätzlich auch andere intermetallische Pulverfraktionen anstelle des beispielhaften $ZrSi_2$ mit ein. Exemplarisch genannt werden insbesondere Dizirkoniumsilizid ($Zr_2Si$) sowie ein Zirkoniumaluminid ($ZrAl_3$). **Fig.9** zeigt die Zeta-Potentiale **12** in [mV] in Abhängigkeit zu den dimensionslosen pH-Werten **13** in analoger Weise zu **Fig.4a.** Wie ersichtlich weisen die vorgenannten intermetallischen Pulverfraktionen (Diirkoniumsilizid, Zirkoniumtrialuminid) grundsätzlich ähnliche Zetapotentialverläufe wie $ZrSi_2$ auf und eigenen sich damit grundsätzlich als intermetallische Verbindung im Rahmen der Erfindung. Dies wurde mit ektrophoretische Abscheidungen verifiziert, wobei die Abscheidungen mit $Zr_2Si$-haltigen Suspensionen (82 Vol.% $Zr_2Si$, 10 Vol.% $SiO_2$, 6 Vol.% $ZrO_2$, 2 Vol.% Spinell) unter Zuhilfenahme eines zitronensäurehaltigen Dispergators problemlos verlief. Dies galt auch für Abscheidungen mit $ZrAl_3$-haltigen Suspensionen (70 Vol.% $ZrAl_3$, 18 Vol.% $ZrSi_2$, 10 Vol.% $SiO_2$, 2 Vol.% Spinell), wobei neben dem zitronensäurehaltigen Dispergator auch ein Natriumdispergiermittel als Dispergator zum Einsatz kam.

Eine Abscheidung $ZrAl_3$-haltiger Suspensionen ist auch in Verbindung mit einer Stabilisierung über den pH-Wert möglich. Allerdings ist zu beobachten, dass die Stabilität der Suspensionen schlechter ist als bei der Verwendung von Dispergatoren, weshalb Dispergatoren einer pH-Stabilisierung vorzuziehen sind. Erfolgreich getestet wurden pH-Werte von 8,9 (intrinsischer pH-Wert ohne Zugabe von Säuren/Basen), 9,7 und 10,9. Mit zunehmendem pH-Wert ist aufgrund der Leitfähigkeitssteigerung eine Abnahme der Abscheiderate zu beobachten.

Die erreichten Gründichten liegen bei der $Zr_2Si$-haltigen Suspension bei etwa 59,1 % theoretischer Dichte und bei den $ZrAl_3$-haltigen Systemen bei 57,1 % (zitronensäurehaltiger Dispergator), 57,9 % (Darvan) bzw. 55,1 % (über pH-Wert stabilisiert). Sie liegen deutlich unterhalb der Gründichten, wie sie mit den untersuchten $ZrSi_2$-haltigen Suspensionen erreicht werden, was letztendlich für das schwindungsfreie Reaktionssintern die Verwendung von Pulvermischungen mit einem deutlich höheren Anteil an intermetallischer Verbindung zur Folge hat.

**Bezugszeichenliste:**

1    Anode
2    Kathode
3    Ausgleichskammer
4    Membran
5    Formfenster
6    Suspensionskammer
7    Volumenanteil in [%]
8    Partikeldurchmesser in [$\mu$m]
9    Viskosität in [Pas]
10    Dispergatoranteil CE-64 in [$\mu$m/m$^2$]
11    Feststoffgehalt in [Vol.%]
12    Zeta-Potential in [mV]
13    ph-Wert [ - ]
14    Gründichten in [% theoretische Dichte]
15    Volumenanteil von $ZrSi_2$ zu $ZrSi_2$ + $ZrO_2$ in [%]
16    Zusammensetzungen (ZrSi2 : ZrO2 : SiO2 : $MgAl_2O_4$), in [Vol.%]

**Patentansprüche**

1. Verfahren zur Herstellung von keramischen Komponenten, umfassend die folgenden Verfahrensschritte:

   a) Bereitstellung einer Dispersion mindestens einer ersten und einer zweiten Pulverfraktion aus einer Oxidkeramik sowie einer dritten Pulverfraktion aus einer intermetallischen Verbindung oder $Zr_2Si$ in einer Flüssigkeit,
   b) Abformung eines Grünkörpers mittels elektrophoretischer Abscheidung aus der Mischung,
   c) Sinterung des Grünkörpers in einer oxidierenden Atmosphäre zu der keramischen Komponente

   wobei

   d) die erste Pulverfraktion durch eine nanoskalige Partikelfraktion mit Partikelgrößen zwischen 2 und 200 nm gebildet wird und als Binder dient,
   e) die zweite Pulverfraktion ein Sinteradditiv umfasst,
   f) der Anteil der dritten Pulverfraktion in Bezug auf die Summe aller Pulverfraktionen einen Volumenanteil zwischen 50 und 95 % einnimmt sowie
   g) die elektrophoretische Abscheidung der Pulverfraktionen zeitgleich erfolgt.

2. Verfahren nach Anspruch 1, **dadurch gekennzeichnet, dass** die Flüssigkeit eine nichtwässrige Lösung ist.

3. Verfahren nach Anspruch 1, **dadurch gekennzeichnet, dass** die Flüssigkeit eine wässrige Lösung ist und in der Dispersion bei der elektrophoretischen Abscheidung ein pH-Wert zwischen 5 und 10 eingestellt wird.

4. Verfahren nach einem der Ansprüche 1 bis 3, **dadurch gekennzeichnet, dass** die Suspension mit einem neutralen Dispergator stabilisiert wird.

5. Verfahren nach einem der vorgenannten Ansprüche, **dadurch gekennzeichnet, dass** die erste und dritte Pulverfraktion eine bimodale Größenverteilung aufweisen.

6. Verfahren nach Anspruch 5, **dadurch gekennzeichnet, dass** die dritte Partikelfraktion Partikelgrößen zwischen 0,2 und 10 $\mu$m aufweist, wobei das Verhältnis der mittleren Partikelgrößen der dritten Partikelfraktionen zu der ersten zwischen 2,5 und 250 liegt.

7. Verfahren nach einem der vorgenannten Ansprüche, **dadurch gekennzeichnet, dass** das Sinteradditiv ein $MgAl_2O_4$-Spinell ist.

8. Verfahren nach einem der vorgenannten Ansprüche, **dadurch gekennzeichnet, dass** der Feststoffanteil der ersten bis dritten Partikelfraktion in der Suspension vor der Abscheidung zwischen 1 und 60 Vol.% beträgt.

9. Verfahren nach einem der vorgenannten Ansprüche, **dadurch gekennzeichnet, dass** die Dispersion weitere Pulverfraktionen aus einem Metall und/oder Oxidkeramik umfasst.

10. Verfahren zur Herstellung von keramischen Dental- oder Mikrokomponenten nach einem der vorgenannten Ansprüche.

Fig.1

Fig.2

Fig.3

9

11

| | 1/s, pH 7,5 |
|---|---|
| | 10/s, pH 7,5 |
| | 100/s, ph 7,5 |
| | 1/s, CE 64 |
| | 10/S, CE 64 |
| | 100/s. CE 64 |

Fig.4a

12

13

Fig.4b

Fig.5

## Fig. 6

## Fig.7

Fig. 8

Fig.9

**Europäisches Patentamt**
**European Patent Office**
**Office européen des brevets**

# EUROPÄISCHER RECHERCHENBERICHT

Nummer der Anmeldung

EP 09 00 5135

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,Y | DE 100 49 974 A1 (WIELAND EDELMETALLE [DE]) 11. April 2002 (2002-04-11) * Ansprüche; Beispiel 1 * ----- | 1,10 | INV. C04B35/622 A61C13/00 A61C13/083 A61K6/02 C25D1/14 C04B35/488 |
| Y | H.-J. RITZHAUPT-KLEISSL ET AL.: "Net Shape Reaction Bonded Ceramic Micro Parts by Mechanical Microstructuring" ADVANCED ENGINEERING MATERIALS, Bd. 8, Nr. 10, 2006, Seiten 983-988, XP002532636 das ganze Dokument, aber insbesonders "Experimental" ----- | 1,10 | |
| Y | GESZWEIN H ET AL: "Fabrication of net shape reaction bonded oxide ceramics" JOURNAL OF THE EUROPEAN CERAMIC SOCIETY, ELSEVIER SCIENCE PUBLISHERS, BARKING, ESSEX, GB, Bd. 26, Nr. 4-5, 1. Januar 2006 (2006-01-01), Seiten 697-702, XP024960553 ISSN: 0955-2219 [gefunden am 2006-01-01] * das ganze Dokument * ----- | 1,10 | |
| P,X | ANDREAS PFRENGLE: "Mikrostrukturierung schwindungsfreier Oxidkeramiken" DISSERTATION, [Online] 31. Juli 2008 (2008-07-31), Seiten 1-177, XP002532637 Gefunden im Internet: URL:http://www.freidok.uni-freiburg.de/vol ltexte/5579/> [gefunden am 2009-06-16] * Seite 29 - Seite 68 * ----- | 1-10 | |

**RECHERCHIERTE SACHGEBIETE (IPC)**

C04B
A61C
A61K
C25D

Der vorliegende Recherchenbericht wurde für alle Patentansprüche erstellt

| Recherchenort | Abschlußdatum der Recherche | Prüfer |
|---|---|---|
| München | 17. Juni 2009 | Munro, Brian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...................................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 09 00 5135

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-06-2009

| Im Recherchenbericht angeführtes Patentdokument | Datum der Veröffentlichung | Mitglied(er) der Patentfamilie | | Datum der Veröffentlichung |
|---|---|---|---|---|
| DE 10049974 A1 | 11-04-2002 | AT | 404154 T | 15-08-2008 |
| | | AU | 1397302 A | 22-04-2002 |
| | | WO | 0230361 A1 | 18-04-2002 |
| | | EP | 1322278 A1 | 02-07-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10049974 A1